Europäisches Patentamt

European Patent Office

Office européen des brevets

(19)

(11) Publication number: **0 273 257**
**A2**

(12) # EUROPEAN PATENT APPLICATION

(21) Application number: **87118236.6**

(22) Date of filing: **09.12.87**

(51) Int. Cl.⁴: **G21K 1/02** , G01T 1/164

(30) Priority: **19.12.86 US 944700**
**25.06.87 US 67059**

(43) Date of publication of application:
**06.07.88 Bulletin 88/27**

(84) Designated Contracting States:
**DE FR GB IT NL**

(71) Applicant: **Siemens Aktiengesellschaft Berlin und München**
**Wittelsbacherplatz 2**
**D-8000 München 2(DE)**

(72) Inventor: **Hsieh, Jiang**
**1124 Lovell Court**
**Elk Grove Village Illinois 60007(US)**

(54) **Scintillation camera and three dimensional multifocal collimator used therewith.**

(57) A collimator (10) has a plurality of focal lengths. The shortest focal length (13) is located at the center (10C) of the collimator. The longest focal length (18) is located at the periphery of the collimator. The focal length increases between the minimum focal length and the maximum focal length.

FIG 1

**Scintillation camera and three dimensional multifocal collimator used therewith**

## BACKGROUND OF THE INVENTION

The invention relates to scintillation cameras, and more particularly relates to scintillation cameras for transaxial ECT (Emission Computed Tomography) imaging. In its most immediate sense the invention relates to tomographic imaging of relatively small body organs such as the heart.

When constructing tomographic images of a small body organ (such as the heart) using transaxial ECT, the scintillation camera head with a collimator attached is rotated around the patient. To prevent the tomographic image from being marred by artifacts, it is necessary to image not merely the heart alone, but the entire slice of the body in which the heart is located. When the collimator used is a fan-beam collimator, the collimator conventionally has a comparatively long focal length, such as 130 centimeters, so that the views of the body are not truncated. This in turn insures that there is complete sampling around the heart and prevents artifacts caused by truncation.

This focal length is appropriate for imaging the periphery of the body, but it inappropriately establishes the sensitivity of the collimator. This is because for fixed resolution and radius of rotation sensitivity decreases as focal length increases. By choosing a single focal length which is just long enough to include the periphery of the body, the sensitivity gain of the collimator is limited by the periphery of the body (which is not generally of interest) rather than by a central body organ of interest (i.e. the heart).

It is therefore on object of the invention to provide a transaxial ECT scintillation camera system which has improved sensitivity in its center without causing truncation errors and corresponding artifacts.

It is another object to provide such a system which will improve images of comparatively small body organs, such as the heart.

It is another object to, in general, improve on known systems of this type.

## SUMMARY OF THE INVENTION

In accordance with the invention, there is provided a scintillation camera system which uses a fan-beam collimator which has a plurality of focal lengths. Advantageously, the collimator focuses to a plurality of focal points which are advantageously colinear and advantageously aligned with a centerline of the collimator. Advantageously, the focal length of the collimator is at a minimum value adjacent a centerline of the collimator and is at a maximum value adjacent peripheral regions of the collimator. Further advantageously, the focal length of the collimator changes (either continuously or stepwise) from the minimum value to the maximum value, and does not decrease with increasing distance from the centerline.

Because the focal length of the collimator is at a minimum in the center of the collimator, there is a large gain in sensitivity in the center of the collimator. Because the focal length at the peripheral regions of the collimator is long enough, the reconstruction artifacts caused by truncation can be avoided.

The invention therefore provides an overall gain in sensitivity in the center of the collimator without any corresponding truncation error at the periphery of the collimator. This permits a better image to be produced in the same period of time, or alternatively permits an equally good image to be produced faster.

## BRIEF DESCRIPTION OF THE DRAWINGS

Exemplary and non-limiting preferred embodiments of the invention are shown in the drawings, in which:

Fig. 1 is a schematic transaxial view of a first preferred embodiment of the invention;

Fig. 2 is a schematic axial view of the first preferred embodiment;

Fig. 3 is a schematic transaxial view of a second preferred embodiment;

Fig. 4 is a diagram illustrating the geometry of the preferred first and second embodiments; and

Fig. 5 is a flowchart of a preferred first method of using the invention;

Fig. 6 is a schematic transaxial view of a third preferred embodiment of the invention;

Fig. 7 is a schematic axial view of the third preferred embodiment;

Fig. 8 is a schematic perspective view of the third preferred embodiment;

Fig. 9 is a schematic axial view of a fourth preferred embodiment;

Fig. 10 is a diagram illustrating the geometry of the preferred third and fourth embodiments; and
Fig. 11 is a flowchart of a preferred second method of using the invention.

DETAILED DESCRIPTION OF PREFERRED EMBODIMENTS

In the following description, it will be assumed that the invention will be used to image a patient's heart. However, it will be understood that this need not be the case and that any other organ can be imaged instead.

To image a patient's heart 2 it is necessary to image a slice 4 of the chest of a patient who is generally indicated by reference numeral 6. This slice 4 is approximately 17 cm in radius. To image this slice 4, a conventional scintillation camera head 8 and attached collimator 10 are rotated along a scan path 12 which is 20 cm in radius.

At the center of the collimator 10, along a centerline 10C thereof, the collimator 10 has a fan-beam configuration with a focal length 13 of 45 cm, i.e. long enough to place the focal point 14 just outside the patient 6. At the peripheral regions 16 of the collimator 10, the focal length 18 is at its maximum, here 130 cm. This focal length 18 is long enough so there is no truncation of any part of the slice 4 because the view of the collimator 10 is slightly wider than the width of the patient 6.

In the first preferred embodiment, the collimator 10 has a focal length which varies continuously with distance; advantageously, the focal length f is determined by the equation:

$$f = R + \cfrac{R}{\left(\cfrac{f_{min}}{f_{min}-R}\right)\left(\cfrac{w-x}{w}\right)^2 + \left(\cfrac{f_{max}}{f_{max}-R}\right)\left(\cfrac{x}{w}\right)^2}$$

Where R = radius of rotation; $f_{min}$ = minimum value of f; $f_{max}$ = maximum value of f, w = half width of collimator; x = distance from center.

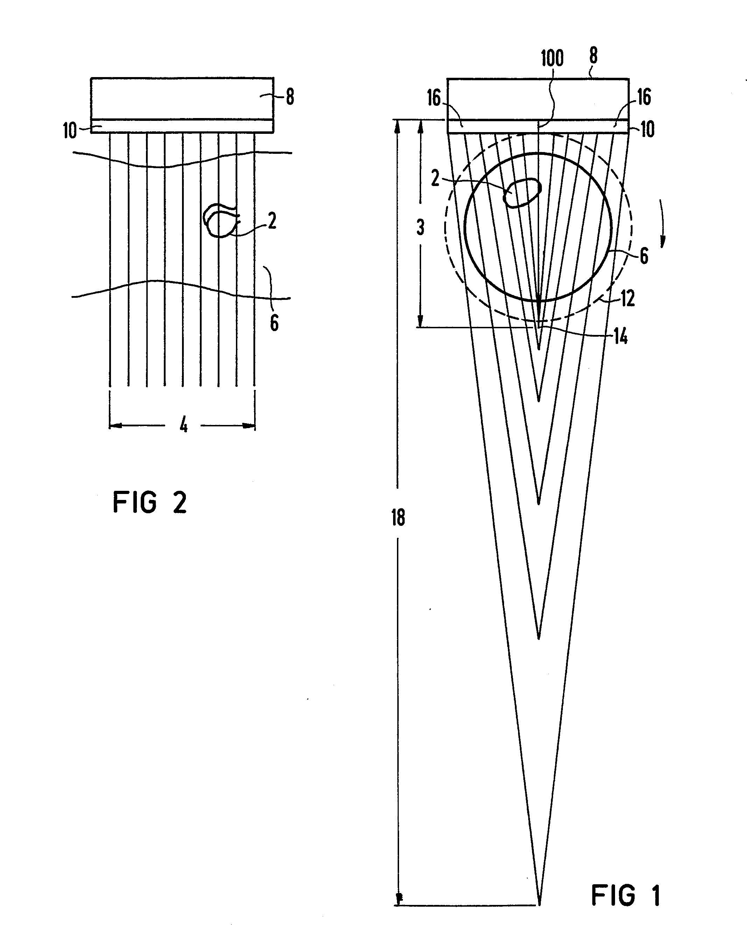

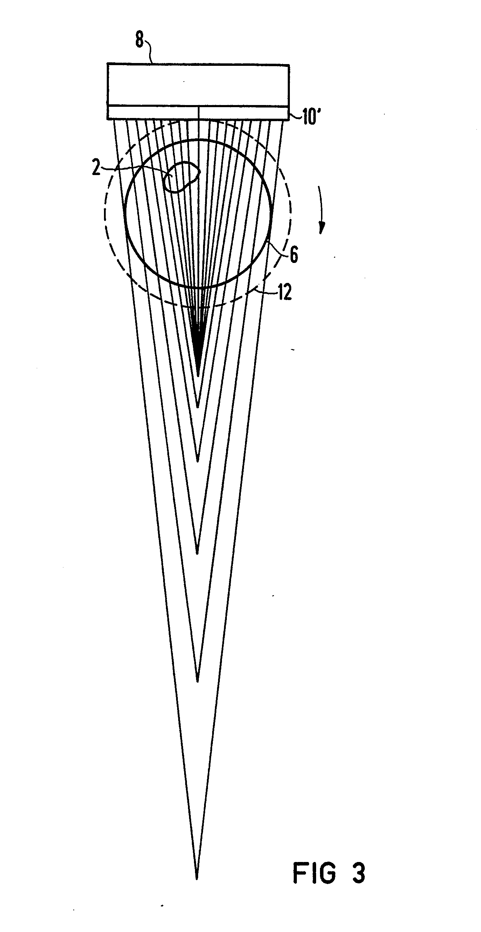

It will be understood that Fig. 1 and 2 are exaggerated for clarity and that there will be hundreds of closely-spaced focal points rather than the six which are shown spaced widely apart.

Alternatively, the focal length may vary discontinuously, e.g. the focal length may be substantially constant over a center region of the collimator and vary over the peripheral regions; this is illustrated in Fig. 3. Fig. 3 is illustrative; there will be more than six focal points, but Fig. 3 has been exaggerated for clarity.

With the focal lengths chosen, the sensitivity of the 10 and 10' collimators at the center are 1.8 times the sensitivity of a parallel-hole collimator of equivalent resolution.

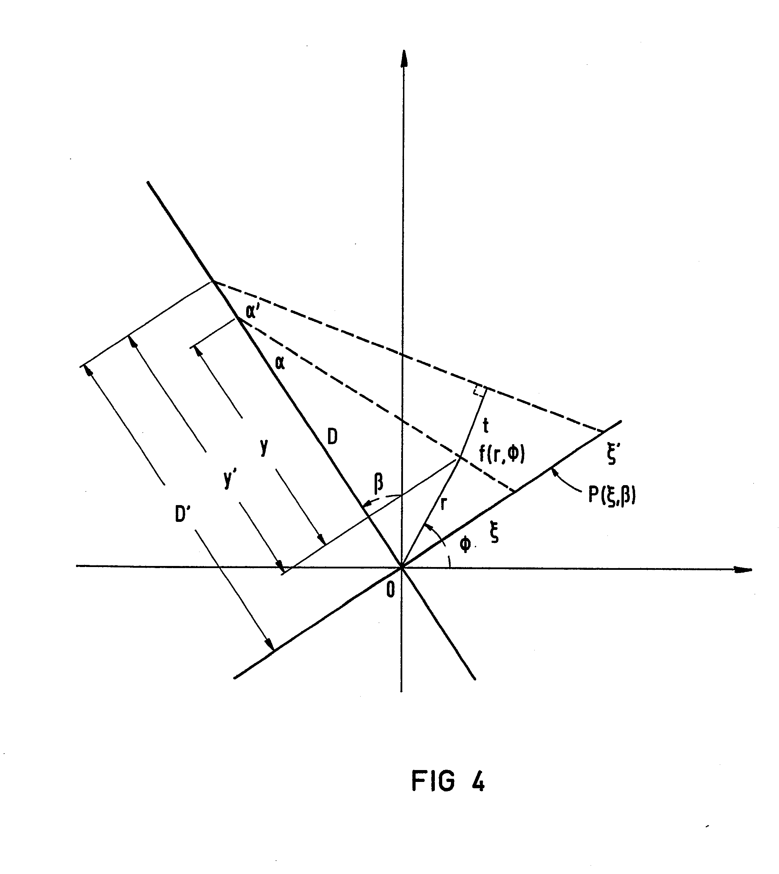

Because the focal length of the collimator varies, the magnification of the image varies as a function of position. To take this into account, the geometrical relationship illustrated in Fig. 4 may be utilized.

In Fig. 4, f(r, $\phi$) represents the point to be reconstructed and P($\xi$, $\beta$) represents the projection image acquired during a single view. A representation of f(r, $\phi$) is given by

$$f(r/\phi) = \frac{1}{4\pi^2} \int_0^{2\pi} g''(\partial, \beta) \frac{D^2}{[D + \gamma \sin(\beta - \phi)]^2} d\beta$$

where g'' ($\partial$, $\beta$) is the filtered and normalized image acquired during the view under consideration and is given by

$$g''(\partial,\beta) = \int_{-\infty}^{\infty} \left(\frac{\xi^2 + D'^2}{D'^2}\right) F_\epsilon \left[\xi - \frac{\sigma}{\sigma'}\xi'\right] \cdot J \cdot P(\xi,\beta)d\varphi$$

where

$$J = \frac{\partial D'}{\partial \xi'}\sin\alpha' + \frac{\partial\alpha'}{\partial\xi'}D'\cos\alpha \quad ; \sigma = \frac{D}{\psi} \quad \sigma' = \frac{D'}{\psi'}$$

and $F_\epsilon$ is the convolution filter.

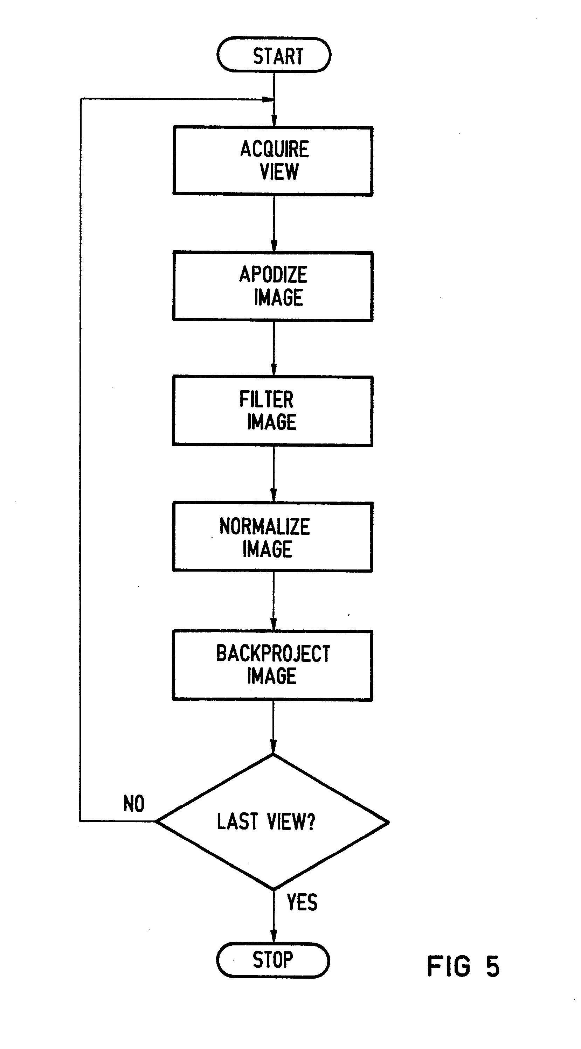

If it is assumed that the convolution filter is spatially invarient, computation speed can be increased, and the image reconstruction process can follow the flow chart shown in Fig. 5.

First, the individual view is acquired. Next, the image is apodized, because apodization is a necessary step whenever a fan beam collimator is used to produce a tomographic image. Next, the apodized image is filtered (deconvoluted), and the apodized and filtered view is normalized to correct for the different magnifications caused by the different focal lengths of the collimator. The apodized, filtered and normalized image is then backprojected into the image space and added to whatever previously processed data is present there. This process is repeated for each view until all views have been acquired. The collimator as disclosed above is a parallel beam collimator as viewed along the axial direction and has its plurality of focal lengths in the transaxial direction. Persons skilled in the art will understand that the term "axial direction" refers to a direction which is parallel to the axis of rotation of the collimator in rotational camera transaxial SPECT (single photon emission computed tomography). "Transaxial direction" means a view taken perpendicular this axis of rotation.

In accordance with another preferred embodiment, there is provided a scintillation camera system which uses a three-dimensional multifocal collimator. As viewed along the transaxial direction the collimator appears identical to the collimator disclosed above. However, in the axial direction, the collimator is a focusing collimator (and not a nonfocusing, parallel-hole collimator). in preferred embodiments, the invention has exactly one, or a plurality, of focal points in the axial direction. The focal lengths of the collimator are at a minimum in the center and at a maximum around the periphery.

The invention provides a further gain in sensitivity because it is a focusing collimator in both the transaxial and axial directions, rather than focusing in the transaxial direction alone. This permits a still better image to be produced in the same period of time, or alternatively permits an equally good image to be produced still faster.

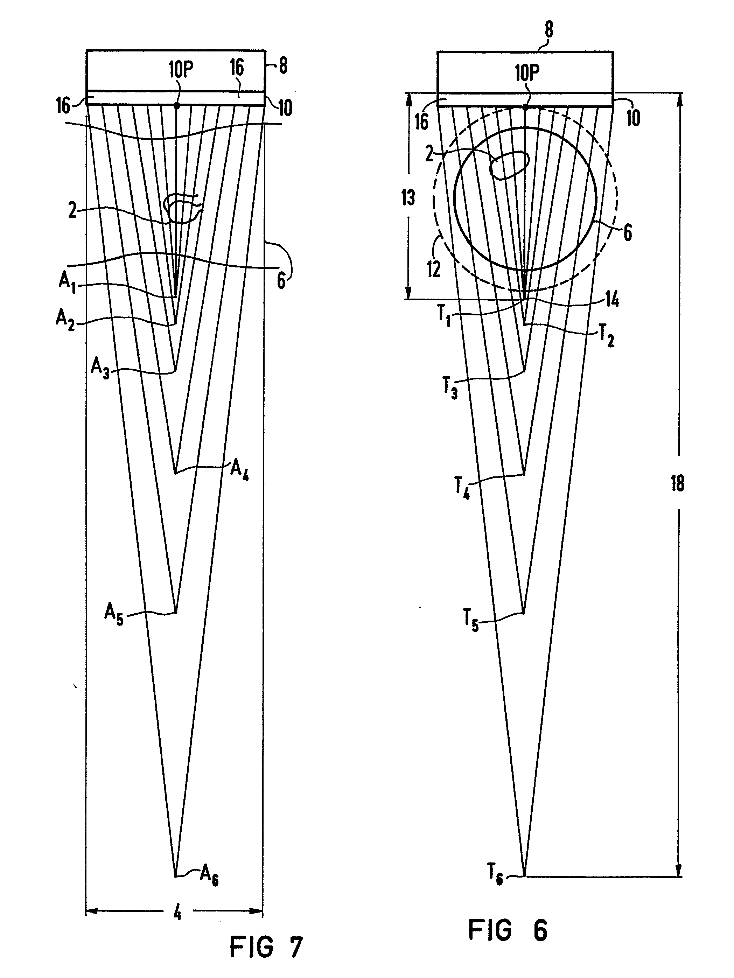

In a third preferred embodiment of the invention, the collimator 10 has a plurality of focal points as viewed transaxially (T₁, T₂ ... etc. in Fig. 6) and axially (A₁, A₂ ... etc. in Fig. 7). In each of the transaxial and axial cases, the focal length of the collimator is at a maximum adjacent the centerpoint 10P of the collimator 10 and at a minimum adjacent the peripheral region 16. The minimum focal length 13 is 45 cm, i.e. long enough to place the focal point 14 just outside the patient 6. The maximum focal length 18 is 130 cm, i.e. long enough so that no part of the slice 4 is truncated because the view of the collimator 10 is slightly wider than the width of the patient 6.

In the third preferred embodiment, the variation of focal length is the same in both the axial and transaxial cases. This is merely for convenience and is not a part of the invention; the focal length variation is the axial view may differ from the focal length variation in the transaxial view. Further, the minimum focal length in the axial case need not be the same as in the transaxial case, and likewise for the maximum focal length.

In the third preferred embodiment, the collimator 10 has a focal length which varies continuously with distance; advantageously, the focal length f is determined by the equation:

$$f(x) = R + \frac{R}{\left(\frac{f_{min}}{f_{min} - R}\right)\left(\frac{w - x}{w}\right)^2 - \frac{f_{max}}{f_{max} - R}\frac{x^2}{w}}$$

where R = radius of rotation

$f_{min}$ = 45 cm (short focal length)

$f_{mx}$ = 150 cm (long focal length)

w = half width of the collimator

x = distance of the point from center of the collimator either in transaxial or axial direction

It will be understood that Figs. 6 and 7 are exaggerated for clarity and that there are actually hundreds of closely spaced focal points rather than the few points which are shown spaced widely apart.

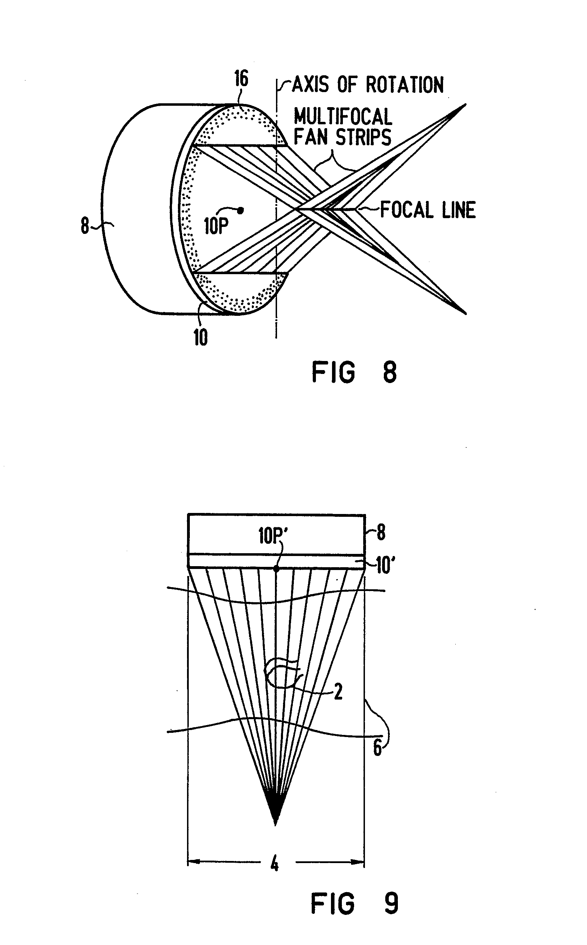

Alternatively, there may be only one focal point in the axial direction; this is illustrated in Fig. 8 and 9.

The third preferred embodiment has a volume sensitivity gain of 2.5 when used for imaging the heart, as compared with the sensitivity of parallel collimators of comparable resolution.

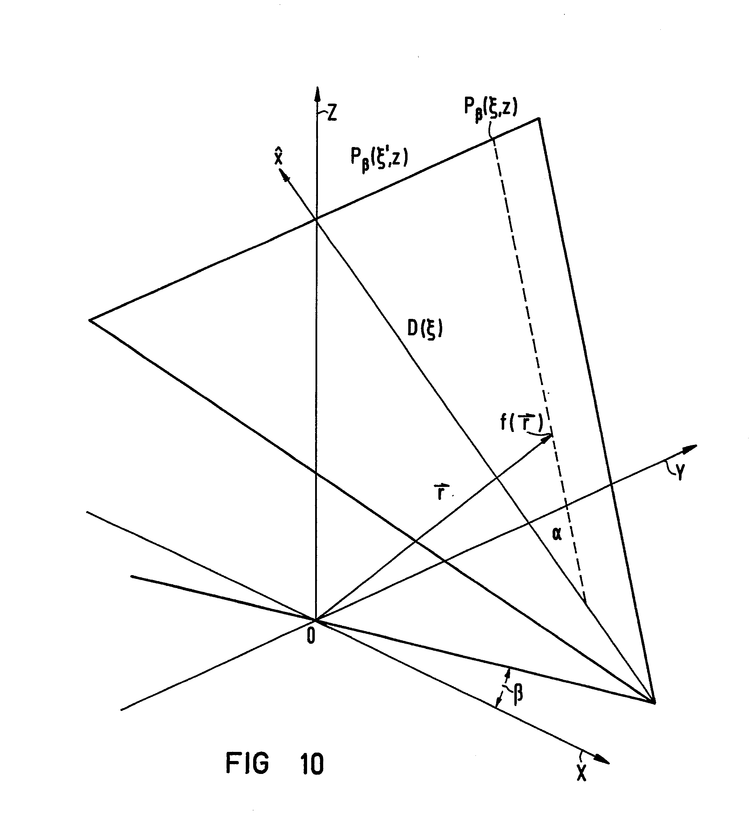

Because the focal length of the collimator varies, the magnification of the image varies as a function of position. To take this into account, the geometrical relationships illustrated in Fig. 10 may be utilized.

In Fig. 10, $f(\vec{r})$ represents the point to be reconstructed and $P_\beta(\xi,z)$ represents the projection image acquired during a single view. A representation of $f(\vec{r})$ is given by

$$f(\vec{r}) = \frac{1}{4\pi^2} \oint g_\beta''(\xi, z) \frac{D^2(\xi)}{[D(\xi) + \vec{r} \cdot \hat{x}]^2} \, d\beta$$

where $g_\beta''(\xi, z)$ is the filtered and normalized image acquired during the view under consideration and is given by

$$g_\beta''(\xi, z) = \int_{-\infty}^{\infty} dz' \int_{-\infty}^{\infty} d\xi' \, g_\xi(\xi - \frac{\sigma}{\sigma'} \xi') \, g_z(z - z') \cdot I \cdot P_\beta(\xi', z)$$

where

$$\sigma = \frac{D(o)}{D(o) + \vec{r} \cdot \hat{x}} \qquad \sigma' = \frac{D(\xi')}{D(\xi') + \vec{r} \cdot \hat{x}}$$

$$I = \frac{\partial D'(\xi)}{\partial \xi'} \cdot \sin \alpha' + \frac{\partial \alpha'}{\partial \xi'} D' \cdot \cos \alpha'$$

and

$$g_\xi(\xi) = R_e \int_0^{\omega_{\xi o}} \omega \, d\omega \exp(i\omega\xi)$$

$$g_z(z) = \frac{\sin \omega_{zo} \cdot z}{\pi z}$$

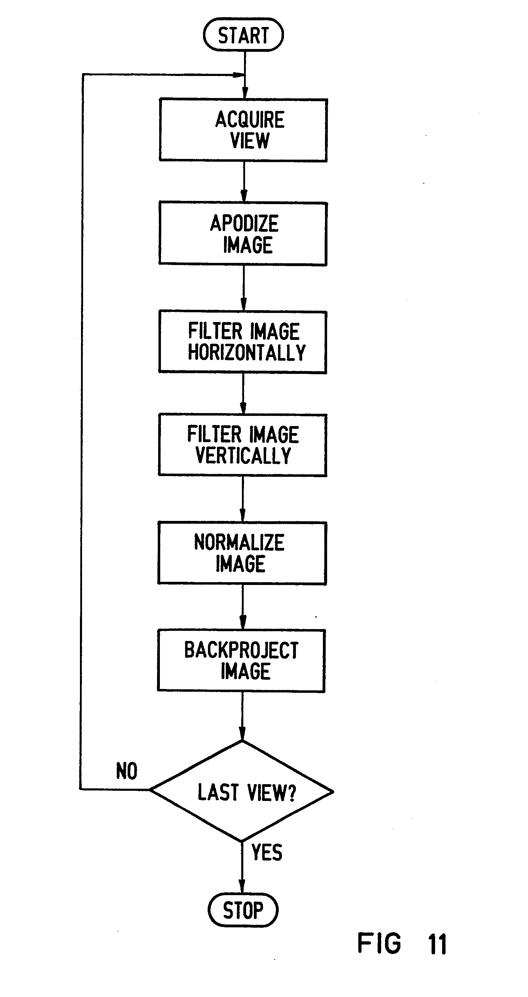

If it is assumed that the convolution filter can be approximated as spatially invariant, computation speed can be increased, and the image reconstruction can follow the flowchart shown in Fig. 11.

First, the individual view is acquired. Next, the image is apodized. Thereafter, the apodized image is filtered, first horizontally, and next vertically, although the order is not part of the invention and vertical filtering can precede horizontal filtering. The apodized and filtered view is normalized to correct for the different magnifications caused by the different focal lengths of the collimator. Finally, the apodized, filtered and normalized image is backprojected into the image space and added to whatever previously processed data is present there. This process is repeated for each view until all views have been acquired.

Those skilled in the art will understand that changes can be made in the preferred embodiments here described, and that these embodiments can be used for other purposes. Such changes and uses are within the scope of the invention, which is limited only by the claims which follow.

## Claims

1. A scintillation camera system, comprising a scintillation camera head (8) and a fan-beam collimator (10) having a plurality of focal lengths (13, 18).

2. A scintillation camera system, comprising a scintillation camera head (8) and a fan-beam collimator (10) which focuses to a plurality of coplanar focal points (14).

3. The scintillation camera system of claim 2, wherein the focal lines are all coplanar with a centerline (10C) of the collimator (10).

4. A fan-beam collimator having a plurality of focal lengths, the focal length (13) of the collimator being at a minimum value adjacent a centerline (10C) of the collimator (10), the focal length (18) of the collimator (10) being at a maximum value adjacent two opposed peripheral regions thereof and the focal length of the collimator nondecreasing from said minimum value to said maximum value as distance from said centerline increases.

5. The collimator of claim 4, wherein the focal length of the collimator (10) varies in discrete steps.

6. The collimator of claim 4, wherein the focal length of the collimator (10) varies continuously.

7. The collimator of claim 4, wherein said minimum value is approximately 45 cm and said maximum value is approximately 130 cm.

8. A scintillation camera system according to claim 1 with a rotational camera transaxial SPECT collimator having a plurality of focal points as viewed along a first direction and at least one focal point as viewed along a second direction which is perpendicular to said first direction.

9. The collimator of claim 8, wherein said first direction is a transaxial direction and wherein said second direction is an axial direction.

10. The collimator of claim 9, wherein there is exactly one focal point as viewed along said axial direction.

11. The collimator of claim 10, wherein said one focal point is at most 130 centimeters distant from a rear face of the collimator.

12. The collimator of claim 8, wherein the collimator has a plurality of focal points as viewed axially and as viewed transaxially.

13. The collimator of claim 12, wherein, as viewed axially and as viewed transaxially, the closest focal point is 45 centimeters distant from a rear face of the collimator and the most remote focal point is 130 centimeters distant from said rear face.

14. A rotational camera transaxial SPECT collimator having a plurality of focal lengths as viewed along an axial direction and as viewed along a transaxial direction, and in which, for focal lengths in each of said axial and transaxial directions,
a minimum focal length exists adjacent a centerpoint of the collimator,
a maximum focal length exists adjacent a periphery of the collimator, and
the focal length of the collimator is nondecreasing from said minimum focal length to said maximum focal length as distance from said centerpoint increases.

15. The collimator of claim 14, wherein said minimum focal length is approximately 45 centimeters and said maximum focal length is approximately 130 centimeters.

16. A rotational camera transaxial SPECT collimator having a single focal length as viewed along an axial direction and a plurality of focal lengths as viewed along a transaxial direction and in which, has focal lengths in said transaxial direction,
a minimum focal length exists adjacent a centerpoint of the collimator,
a maximum focal length exists adjacent a periphery of the collimator, and
the focal length of the collimtor is nondecreasing from said minimum focal length to said maximum focal length as distance from said centerpoint increases.

17. The collimator of claim 16, wherein said minimum focal length is approximately 45 centimeters and said maximum focal length is approximately 130 centimeters.

18. A rotational camera transaxial SPECT scintillation camera system, comprising:
a scintillation camera head;
a gantry supporting the scintillation camera head for use in rotational camera transaxial SPECT; and
a collimator having a plurality of focal points as viewed along a first direction and at least one focal point as viewed along a second direction which is perpendicular to said first direction.

19. The system of claim 18, wherein said first direction is a transaxial direction, said second direction is an axial direction, and wherein there is exactly one focal point as viewed from said axial direction.

20. The system of claim 18, wherein said first direction is a transaxial direction, said second direction is an axial direction, and wherein there are a plurality of focal points as viewed from said axial direction.

86 P 7430 E

FIG 2

FIG 1

86 P 7430 E

FIG 3

FIG 4

86 P 7430 E

```
                    ┌─────────┐
                    │  START  │
                    └─────────┘
                         │
      ┌──────────────────┼
      │                  ▼
      │            ┌─────────────┐
      │            │   ACQUIRE   │
      │            │    VIEW     │
      │            └─────────────┘
      │                  │
      │                  ▼
      │            ┌─────────────┐
      │            │   APODIZE   │
      │            │    IMAGE    │
      │            └─────────────┘
      │                  │
      │                  ▼
      │            ┌─────────────┐
      │            │   FILTER    │
      │            │    IMAGE    │
      │            └─────────────┘
      │                  │
      │                  ▼
      │            ┌─────────────┐
      │            │  NORMALIZE  │
      │            │    IMAGE    │
      │            └─────────────┘
      │                  │
      │                  ▼
      │            ┌─────────────┐
      │            │ BACKPROJECT │
      │            │    IMAGE    │
      │            └─────────────┘
      │                  │
      │                  ▼
      │       NO    ◇─────────────◇
      └────────────│  LAST VIEW?  │
                   ◇─────────────◇
                         │ YES
                         ▼
                    ┌─────────┐
                    │  STOP   │
                    └─────────┘
```

FIG 5

FIG 7

FIG 6

**FIG 8**

16
AXIS OF ROTATION
MULTIFOCAL FAN STRIPS
FOCAL LINE
8
10P
10

**FIG 9**

10P'
8
10'
2
6
4

FIG 10

FIG 11